# EUROPEAN PATENT SPECIFICATION

(11) **EP 0 746 286 B1**
(45) Date of publication and mention of the grant of the patent: **08.04.1998**
(21) Application number: 92915258.5
(22) Date of filing: 26.06.1992
(51) Int. Cl.: A61F 5/40, A41B 9/02

(54) **TESTICLE SUPPORT GARMENT**
DIE HODEN STÜTZENDES BEKLEIDUNGSSTÜCK
VETEMENT DE SUPPORT POUR LES TESTICULES

(43) Date of publication of application: 11.12.1996
(73) Proprietor: RUDKIN, William, Old Lyme, CT 06371 (US)
(72) Inventor: Simmons, Gregory C., Shelbourne, Vermont 05482 (US)
(74) Representative: Effert, Udo, Dipl.-Ing.
(86) International application number: US9205415
(87) International publication number: WO9400084

(56) References cited:
- DE-C- 889 676
- US-A- 4 487 202
- US-A- 4 955 088
- US-A- 5 131 386

## Description

### FIELD OF THE INVENTION

This invention pertains to testicle support means, and in particular to such testicle support means provided by wearing apparel, specifically wearing apparel that covers the crotch portion and passes between the legs of the user.

### BACKGROUND OF THE INVENTION

Testicle support is well known and a necessary function in athletic and medical situations. It is known in the art that a testicle support comprises two important processes. The first is to elevate or pull the user's testicles forward and out from between the user's legs and the second is to hold the testicles in place. The prior art uses these processes either individually or in conjunction.

Examples of these type devices include United States Patent No. 2,294,066 issued to Baehler on August 25, 1942 ("Baehler"), for a "Suspensory" and United States Patent No. 4,487,202 issued to Sachse on December 11, 1984 ("Sachse"), for "Testis Support For Elevating and Treating Diseased Testes and Epididymides". The general function of these references and a number of others in the art is to get behind and under the testes and scrotum, to move the testes forward and out from between the legs and to anchor the testes out of the way. The Baehler reference uses loops to accomplish this goal. However, these references are limited as to the garment they could be used with and also as to that it would be fairly uncomfortable to be used in everyday situations.

Clearly, it is desirable to have a testicle support means and a method of providing testicle support that can be used comfortably in all situations and with a number of different garments. It is the object of this invention, then to set forth a testicle support means which avoids the disadvantages and limitations, above-recited, which were obtained in prior testicle supports. It is also the object of this invention to teach a testicle support means that can be positioned or sewn into any men's garments to be worn directly next to the skin in the crotch area. Another object of this invention is to teach a novel means of contacting the perineum and the posterior portion of the user's scrotum and testicles in order to provide a means for testicle support.

### SUMMARY OF THE INVENTION

Particularly, it is the object of this invention to set forth a testicle support means, for use in men's garments worn next to the skin, comprising a male garment. The male garment comprises a first means for surrounding the lower trunk of the user's body, a second means for surrounding at least part of each of the user's legs separately, and a connection means. The connection means comprises fabric material for covering the crotch portion of the user and for connecting the first means and the second means. The connection means has a pad support means which comprises a crescent-shaped form of flexible material attached to the connection means for contacting the perineum and the posterior portion of the user's scrotum. The crescent-shaped form has a center section and the crescent-shaped form has twin end sections. The crescent-shaped form further has a center section with a thicker cross-sectional dimension, and twin end sections having a thinner cross-sectional dimension.

Further objects of this invention will become more apparent by reference to the following description taken in conjunction with the accompanying figures.

### BRIEF DESCRIPTION OF THE DRAWINGS

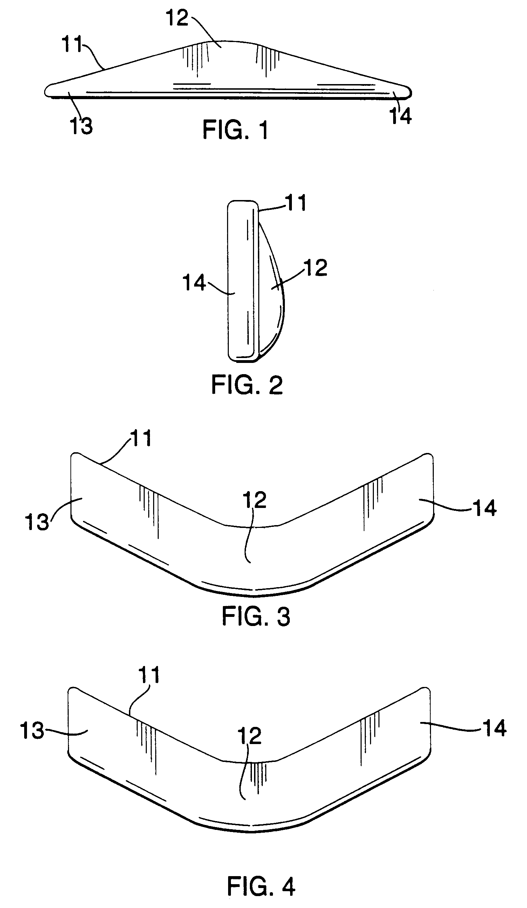

Fig. 1 is an frontal view of the novel testicle support means;
Fig. 2 is a side view thereof;
Fig. 3 is a top view thereof;
Fig. 4 is a bottom view thereof;

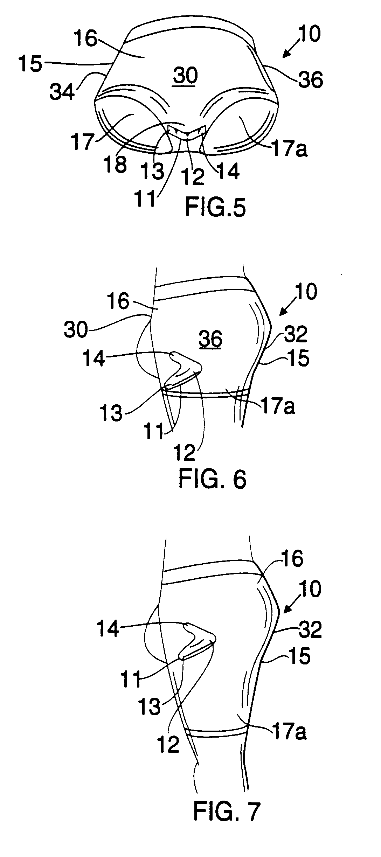

Fig. 5 is a perspective view of the novel support means in position in men's briefs;
Fig. 6 is a side perspective view of the novel support means in position behind the user's scrotum in men's briefs; and
Fig. 7 is a side elevational view of the novel support means in position in long leg apparel.

### DETAILED DESCRIPTION OF THE PREFERRED EMBODIMENT

As shown in the Figures, the testicle support means (such as a cushion) 10 comprises an insert 11 that is formed in a crescent or winged shape. The insert 11 has a thickened center section 12 and twin end sections 13 and 14. The insert is cut from a flexible foam material, or the like, to a predetermined shape. The insert 11 is then positioned in men's briefs 15, shorts, a swimsuit, or the like. The garment 15 has a trunk covering or encircling portion 16, having a front 30, a back 32, and two sides 34 and 36. A connection piece or crotch panel 18 connects the front and back of the trunk covering portion 16 and with sides 34 and 36, defines the leg covering portions 17 and 17a of the garment 15. The insert 11 is attached to the connection piece or crotch panel 18. The insert 11 is sewn or glued into position in the garment 15 in the crotch area 18, so that when the garment is worn the insert will be located behind the male's testicles, so that the small vacant area behind the testicles is filled by the insert 11. The insert 11 is adapted to straddle and be in contact with the posterior and lateral portions of the user's scrotum and testicles. The center section 12 of the insert 11 is aligned with the midsagittal plane of the user's body and the twin end sections 13 and 14 are tapered down from the center section 12 to be angled forward toward the front of the user's body and have sufficient length to be adjacent to the user's thighs. The insert 11 is positioned in the garment 15 so that it passes between the user's legs forward of the coronal plane of the user's body. This positioning allows the structure to elevate the user's testicles forward and out from between the legs thus allowing the apparel to hold the testicles in place. A material covering is provided for the insert 11 to facilitate the ease of attaching the insert into the garment 15.

While I have described my invention in connection with specific embodiments thereof, it is clearly to be understood that this is done only by way of example and not as a limitation to the scope of my invention as set forth in the appended claims.

## Claims

1. A garment (15) to be worn next to the skin of a male user's testicles, said garment comprising:
a body portion (16) for encircling said user's body trunk, said body portion having a front (30), a back (32) and two sides (34, 36);
a crotch panel (18) extending between said front (30) and said back (32) of said body portion for connecting them, said crotch panel and the sides of said body portion defining therebetween two leg openings (17, 17a), each of said openings being defined by a side edge of said crotch panel (18) and the adjacent side of said body portion;
a support means (10) comprising a cushion secured to said crotch panel (18) in such a position and extending in such a direction so that when worn by said user, said cushion extends inwardly of the garment from the crotch panel (18) and upwardly therefrom for engaging a portion of said user's perineum and a portion of the rear of said user's scrotum for positioning the user's testicles away from a position between the user's legs.

2. The garment of claim 1, wherein said crotch panel comprises a fabric material.

3. The garment of claim 1, wherein said support means is connected to said crotch panel by adhesive.

4. The garment of claim 1, wherein said support means is connected to said crotch panel by a sewn seam.

5. The garment of claim 1, further comprising a covering material, wherein said cushion is covered by said covering material.

6. The garment of claim 1, wherein said cushion is crescent-shaped.

7. The garment of claim 6, wherein said crescent-shaped cushion includes a center section (12) of a given thickness and twin end sections (13, 14) of less thickness than said center section (12).

8. The garment of claim 6, wherein said center section of said crescent-shaped cushion is aligned with the midsagittal plane of said body portion, said twin end sections (13, 14) are tapered in thickness from said center section (12) and are angled forward from said center section (12) toward the front (30) of said body portion and having sufficient length to be adjacent to the user's legs.

9. The garment of claim 6 or 8, wherein said crescent-shaped cushion is made of a flexible foam material.

## Patentansprüche

1. Bekleidungsstück (15), das unmittelbar auf der Haut der Hoden eines männlichen Benutzers zu tragen ist, umfassend:
ein Hauptteil (16) zum Umschlingen des Rumpfes des Benutzers, das eine Vorderseite (30), eine Rückseite (32) und zwei Seiten (34, 36) aufweist;
einen Schritteinsatz (18), der sich zwischen der Vorderseite (30) und der Rückseite (32) des Hauptteils erstreckt, um sie zu verbinden, wobei der Schritteinsatz und die Seiten des Hauptteils dazwischenliegend zwei Beinöffnungen (17, 17a) bilden, und jede der Öffnungen durch eine Seitenkante des Schritteinsatzes (18) und die angrenzende Seite des Hauptteils gebildet ist;
eine Stützeinrichtung (10) mit einem Polster, das mit dem Schritteinsatz (18) an einer solchen Position befestigt ist und sich in einer solchen Richtung erstreckt, so daß beim Tragen durch den Benutzer das Polster im Inneren des Bekleidungsstückes aus dem Schritteinsatz (18) und von dort nach oben verläuft zum Einbinden eines Teils des Dammes und eines Teils vom hinteren Teil des Hodensacks des Benutzers, um die Hoden des Benutzers entfernt von einer Lage zwischen dessen Beinen zu positionieren.

2. Bekleidungsstück nach Anspruch 1, wobei der Schritteinsatz Gewebematerial enthält.

3. Bekleidungsstück nach Anspruch 1, wobei die Stützeinrichtung mit dem Schritteinsatz durch Klebstoff verbunden ist.

4. Bekleidungsstück nach Anspruch 1, wobei die Stützeinrichtung durch eine genähte Naht mit dem Schritteinsatz verbunden ist.

5. Bekleidungsstück nach Anspruch 1, das ferner ein Bezugsmaterial aufweist, wobei das Polster mit diesem Bezugsmaterial bezogen ist.

6. Bekleidungsstück nach Anspruch 1, wobei das Polster halbmondförmig ist.

7. Bekleidungsstück nach Anspruch 6, wobei das halbmondförmige Polster einen mittleren Abschnitt (12) mit vorgegebener Dicke und Doppelendabschnitte (13, 14) mit einer geringeren Dicke als der des mittleren Abschnitts (12) einschließt.

8. Bekleidungsstück nach Anspruch 6, wobei der mittlere Abschnitt des halbmondförmigen Polsters zur mittleren sagittalen Ebene des Hauptteils ausgerichtet ist, die Doppelendabschnitte (13, 14) von dem mittleren Abschnitt (12) in der Dicke verjüngt und von diesem mittleren Abschnitt (12) nach vorn zur Vorderseite (30) des Hauptteils hin winklig sind und eine ausreichende Länge aufweisen, um an den Beinen des Benutzers anzugrenzen.

9. Bekleidungsstück nach Anspruch 6 oder 8, wobei das halbmondförmige Polster aus einem nachgiebigen Schaumstoff hergestellt ist.

## Revendications

1. Vêtement (15) destiné à être porté proche de la peau des testicules d'un utilisateur mâle, comprenant :
une partie principale (16) pour entourer le tronc du corps dudit utilisateur, ladite partie principale ayant un avant (30), un arrière (32) et deux côtés (34,36);
un panneau d'entre-jambes (18) s'étendant entre ledit avant (30) et ledit arrière (32) de ladite partie principale de façon à les lier, ledit panneau d'entre-jambes et les côtés de ladite partie principale définissant entre eux deux ouvertures pour les jambes (17, 17a), chacunes desdites ouvertures étant définie par un bord de côté dudit panneau d'entre-jambes (18) et par le côté adjacent de ladite partie principale;
un moyen de support (10) comprenant un coussin fixé audit panneau d'entre-jambes (18) dans une telle position et s'étendant dans une telle direction afin que lorsque qu'il est porté par ledit utilisateur, ledit coussin s'étend intérieurement dans le vêtement à partir du panneau d'entre-jambes (18) et vers le haut à partir de celui-ci pour venir prendre une partie du périnée dudit utilisateur et une partie de l'arrière du scrotum dudit utilisateur pour positionner les testicules de l'utilisateur en dehors d'une position située entre les jambes de l'utilisateur.

2. Vêtement selon la revendication 1, dans lequel ledit panneau d'entre-jambes est composé de tissu.

3. Vêtement selon la revendication 1, dans lequel ledit moyen de support est relié audit panneau d'entre-jambes par adhésif.

4. Vêtement selon la revendication 1, dans lequel ledit moyen de support est relié audit panneau d'entre-jambes par une couture cousue.

5. Vêtement selon la revendication 1, comprenant en outre un matériau enveloppant, dans lequel ledit coussin est enveloppé par ledit matériau enveloppant.

6. Vêtement selon la revendication 1, dans lequel ledit coussin adopte la forme d'un croissant.

7. Vêtement selon la revendication 6, dans lequel ledit coussin adoptant la forme d'un croissant comprend une section centrale (12) d'une épaisseur donnée et des sections extrèmes jumelles (13,14) d'épaisseur moindre que celle de ladite section centrale (12).

8. Vêtement selon la revendication 6, dans lequel ladite section centrale dudit coussin adoptant la forme d'un croissant est aligné avec le demi-plan vertical de ladite partie principale, lesdites sections extrèmes jumelles (13, 14) sont fuselées dans leur épaisseur à partir de la section centrale (12) et sont dirigées vers l'avant à partir de ladite section centrale (12) vers l'avant (30) de ladite partie principale et ayant une longueur suffisante pour être adjacent aux jambes de l'utilisateur.

9. Vêtement selon la revendication 6 ou 8, dans lequel ledit coussin adoptant la forme d'un croissant est réalisé dans un matériau mousse flexible.
